# EUROPÄISCHE PATENTANMELDUNG

(11) **EP 2 019 085 A1**
(43) Veröffentlichungstag der Anmeldung: **28.01.2009**
(21) Anmeldenummer: 07109131.8
(22) Anmeldetag: 29.05.2007
(51) Int. Cl.: C01G 1/02, C01G 9/02, C09C 1/04

(54) **Verfahren zur Herstellung von Zinkoxiddispersionen zur Verwendung in kosmetischen Zubereitungen**

(71) Anmelder: BASF SE, 67056 Ludwigshafen (DE)
(72) Erfinder: Die Erfindernennung liegt noch nicht vor

(57) **Zusammenfassung**

Die vorliegende Erfindung betrifft ein Verfahren zur Herstellung von Metalloxid enthaltenden kosmetischen Zubereitungen, wobei die bei der Herstellung des partikulären Metalloxids entstehende Reaktionsmischung im Wesentlichen ohne weitere Aufarbeitung in die kosmetischen Zubereitungen eingebracht wird.

## Beschreibung

Die vorliegende Erfindung betrifft ein Verfahren zur Herstellung von Metalloxid enthaltenden kosmetischen Zubereitungen, wobei die bei der Herstellung des partikulären Metalloxids entstehende Reaktionsmischung im Wesentlichen ohne weitere Aufarbeitung in die kosmetischen Zubereitungen eingebracht wird.

Mineralische Pigmente, die Licht durch Absorption, Reflexion und Streuung abschwächen, dienen als physikalische UV-Filter. Man unterscheidet Makropigmente mit einer Partikelgröße über 100 nm und Mikropigmente mit einer Partikelgröße unter 100 nm. Titandioxid, Zinkoxid, Eisenoxide, Kalziumkarbonat, Kaolin und Talkum werden als Suspension eines Pigmentpulvers in Lichtschutzmitteln verwendet. Das Deckungsvermögen ist abhängig vom Verhältnis der Brechzahlen des Pigments und des umgebenden Mediums, dem Ausmaß der Lichtabsorption sowie der Wellenlänge des einfallenden Lichtes und der Partikelgröße. Obwohl mit hohen Konzentrationen von Pigmenten ein totaler Schutz im Bereich des UV-Lichtes sowie des sichtbaren Lichtes erzielbar ist, ist die Weißfärbung der Haut nachteilig und wird häufig als kosmetisch störend empfunden. Mittels moderner Technologien können Titan- und Zinkoxide auf kosmetisch akzeptable Partikelgrößen verkleinert werden. Hierdurch wird die Weißfärbung der Haut vermieden. Ultrafeines Titandioxid und Zinkoxid sind derzeit die wichtigsten im kosmetischen Lichtschutzbereich verwendeten mineralischen Lichtschutzstoffe.

Die Herstellung von Metalloxid-Suspensionen durch Solvolyse (Hydrolyse) von geeigneten Vorstufen in organischen Lösungsmitteln ist seit langem bekannt.

US 4,410,446 beschreibt die Herstellung von stabilen Zinkoxid-haltigen Suspensionen durch Erhitzen von Zinkacetat in einer schwerflüchtigen inerten Flüssigkeit. Als Dispergierhilfsmittel wird Magnesiumnaphthenat verwendet.

US 4,193,769 beschreibt die Herstellung von stabilen Zinkoxid-haltigen Suspensionen durch Erhitzen von Zinkcarbonat in einer schwerflüchtigen inerten Flüssigkeit. Als Dispergierhilfsmittel werden ungesättigte Fettsäuren, Sulfonsäure, oxalkylierte langkettige Amine etc. verwendet.

DE 102 97 544 beschreibt Metalloxid-Dispersion, umfassend ein Metalloxid mit einem Teilchendurchmesser von weniger als 200 nm und ein Dispergiermedium, wobei das Metalloxid-Dispergiermedium einen mehrwertigen Alkohol und/oder eine Polyether-Verbindung umfasst. Die so erhaltenen Dispersionen werden zur Herstellung von Metall-Dünnfilmen auf Substraten verwendet.

JP 2003268368 beschreibt einen UV-Emitter der Zinkoxid-Partikel umfasst. Die Zinkoxid-Partikel sind frei von Alkalimetallen sowie Halogeniden und werden durch Erhitzen einer Mischung aus Zn-Carboxylaten (z. B. Zn-Formiaten, -Acetaten, -Oxalaten, - Adipaten, -Terephthalaten) und Alkoholen (z. B. Methanol, Ethanol, Ethylenglykol, 1,4-Butandiol, Polyethylenglykol) bei ca. 100 - 300 °C hergestellt. Eine mögliche Verwendung dieser Zinkoxid-Partikel in der Kosmetik wird erwähnt.

JP 07-232919 beschreibt ein Verfahren zur Herstellung von Zinkoxid-Partikeln, wobei diese Zinkoxid-Partikel durch Erhitzen einer Mischung aus Zink bzw. einer Zn-Verbindung (z. B. Zinkoxid, Zinkhydroxid, Zinkhydroxidcarbonat, Zinkacetat), einer Verbindung mit zumindest einer Carboxylgruppe (z. B. Ameisensäure, Oxalsäure, Maleinsäure, Terephthalsäure) und Alkoholen (z. B. Methanol, Ethanol, Ethylenglykol, 1,4-Butandiol, Polyethylenglykol) bei ca. 100 - 300 °C hergestellt werden.

Feldmann (Adv. Funct. Mater. 2003, 13, No. 2, February) beschreibt die Herstellung von nanopartikulären Metalloxiden durch Thermolyse geeigneter Vorstufen in Diethylenglykol (sog. Polyol-Methode).

Jézéquel et al. (J. Mater. Res. Vol. 10, No.1, Jan 1995) beschreiben die Herstellung von monodispersen, sphärischen Zinkoxid-Partikeln mit Durchmessern von 0,2 bis 0,4 Mikrometern durch Hydrolyse von Zinkacetat-Dihydrat in Diethylenglykol.

E. Hosono et al. (J. Sol-Gel Sci.Techn. 2004, 29, 71-79) beschreiben die Herstellung sphärischer, monodisperser ZnO-Partikel mit einem Durchmesser von 5 bis 10 nm durch Erhitzen von Zn-Acetat bei 60°C in Methanol, Ethanol und 2-Methoxyethanol. Die Produkte enthalten häufig Verunreinigungen an Zinkhydroxyacetaten.

Collins et al. (J. Mat. Chem. 1992, 2 (12), 1277-1281) beschreiben die Herstellung von CeO₂, ZrO₂ und ZnO durch thermische Zersetzung von den entsprechenden löslichen Ce-, Zr- und Zn-Salzen (Ce-, Zr-, Zn-Nitrate, Zr-Iodid oder Zn-Acetat) in 1-Decanol, 1-Undecanol und Ethylenglykol bei ca. 200 °C. Auf diesem Weg werden monodisperse, sphärische Partikel mit einem Durchmesser ca. 0,25 Mikrometer hergestellt.

Partikelförmiges Zinkoxid ist als UV-Lichtschutzmittel in kosmetischen Zubereitungen seit langem bekannt. Kommerzielle Produkte sind beispielsweise unter den Marken Z-Cote^{®} (BASF), Creazinc^{®} (Creations Couleurs), Finex-25^{®} (Presperse, Inc.), NanoGard Zinc Oxide^{®} (Nanohybrid Co., LTD), Nano-Zinc^{®}SL (Sino Lion (USA) Ltd.), OriStar^{®}ZO (Orient Stars LLC), Oxyde de Zinc Micropure^{®} (LCW - Sensient Cosmetic Technologies), Tego Sun^{®} Z 500(Degussa Care & Surface Specialties), Unichem^{®}ZO (Universal Preserv-A-Chem, Inc.), USP-1^{®} (Zinc Corporation of America) oder Zinc Oxide NDM^{®}106407 (Symrise) erhältlich.

Die durch Zersetzung von Metallsalzen in Suspension hergestellten mikropartikulären Metalloxide werden üblicherweise getrocknet und in Pulverform überführt. Beim Redispergieren in kosmetisch akzeptablen Flüssigkeiten wie beispielsweise Alkoholen geht die Feinteiligkeit verloren, es kommt vielmehr zur Aggregation und Bildung größerer Teilchen, die dann nicht mehr die gewünschten Effekte in den kosmetischen Zubereitungen haben und beim Auftragen auf die Haut zu einer verstärkten Weißfärbung führen.

Eine Aufgabe der vorliegenden Erfindung war es deshalb, ein wirtschaftliches Verfahren bereitzustellen, das es erlaubt, mikropartikuläre Metalloxide möglichst ohne Aggregation in kosmetische Zubereitungen einzubringen.

Gelöst wurde diese Aufgabe durch ein Verfahren zur Herstellung von Metalloxid enthaltenden kosmetischen Zubereitungen umfassend wenigstens folgende Schritte:
a) Herstellung des Metalloxids durch Umsetzung einer geeigneten Vorstufe in einer Alkohol umfassenden Reaktionsmischung,
b) gegebenenfalls Entfernen von bis zu 90 Gew.-% der flüchtigen Bestandteile der aus Schritt a) erhaltenen Metalloxid-Reaktionsmischung,
c) gegebenenfalls wenigstens teilweises Austauschen der flüssigen Phase 1 der Reaktionsmischung gegen eine von der flüssigen Phase 1 unterschiedliche flüssige Phase 2,
d) Verwendung der nach den Schritten a), gegebenenfalls b) und gegebenenfalls c) erhaltenen Reaktionsmischung zur Herstellung der kosmetischen Zubereitung.

### Schritt a)

Die Herstellung von feinteiligen Metalloxiden durch Umsetzung einer geeigneten Vorstufe in einer Alkohol umfassenden Reaktionsmischung ist dem Fachmann bekannt und in den oben genannten Textstellen beschrieben, worauf hiermit in vollem Umfang Bezug genommen wird.
Bevorzugtes Metalloxid der vorliegenden Erfindung ist Zinkoxid. Die Herstellung von Zinkoxid-Partikeln durch Erhitzen von Zinkacetatdihydrat in Diethylenglykol ist dem Fachmann bekannt und beispielsweise beschrieben in Jezequel et al., Volumes 152-153 of Materials Science Forum, ISSN 0255-5476, S.339-342 und Jezequel et al., J. Mater. Res. 1994, 10, 77, worauf hiermit in vollem Umfang Bezug genommen wird. Erfindungsgemäß geeignete Alkohole besitzen eine, bevorzugt wenigstens zwei OH-Gruppen pro Molekül.

Geeignete einwertige Alkohole sind ausgewählt aus Methanol, Ethanol, n-Propanol, i-Propanol, n-Butanol, i-Butanol, sec-Butanol, t-Butanol, n-Pentanol, i-Pentanol, 2-Methylbutanol, sec-Pentanol, t-Pentanol, 3-Methoxybutanol, n-Hexanol, 2-Methylpentanol, sec-Hexanol, 2-Ethylbutanol, sec-Heptanol, 3-Heptanol, n-Octanol, 2-Ethylhexanol, sec-Octanol, n-Nonylalkohol, 2,6-Dimethylheptanol-4, n-Decanol, sec-Undecylalkohol, Trimethylnonylalkohol, sec-Tetradecylalkohol, sec-Heptadecylalkohol, Phenol, Cyclohexanol, Methylcyclohexanol, 3,3,5-Trimethylcyclohexanol, Benzylalkohol und Diacetonalkohol;
Weitere geeignete einwertige Alkohole sind ausgewählt aus partiellen Ethern von Glykolen wie Ethylenglykolmonomethylether, Ethylenglykolmonoethylether, Ethylenglykolmonopropylether, Ethylenglykolmonobutylether, Ethylenglykolmonohexylether, Ethylenglykolmonophenylether, Ethylenglykolmono-2-ethylbutylether, Diethylenglykolmonomethylether, Diethylenglykolmonoethylether, Diethylenglykolmonopropylether, Diethylenglykolmonobutylether, Diethylenglykolmonohexylether, Propylenglykolmonomethylether, Propylenglykolmonoethylether, Propylenglykolmonopropylether, Propylenglykolmonobutylether, Dipropylenglykolmonomethylether, Dipropylenglykolmonoethylether und Dipropylenglykolmonopropylether.

Bevorzugt ist die Verwendung von Alkoholen mit wenigstens zwei OH-Gruppen. Geeignete mehrwertige Alkohole sind beispielsweise Diole. Diese sind bevorzugt ausgewählt aus 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 2,4-Pentandiol, 2-Methyl-2,4-pentandiol, 1,2-Hexandiol, 1,6-Hexandiol, 2,5-Hexandiol, 2,4-Heptandiol, 2-Ethyl-1,3-hexandiol, Octandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Dipropylenglykol, Triethylenglykol, Tetraethylenglykol, Tripropylenglykol, Tetrapropylenglykol, 3-Thio-pentan-1,5-diol, Polyethylenglykole, Polypropylenglykole und Polytetrahydrofurane mit Molekulargewichten von jeweils 200 bis 10000, Diole auf Basis von Blockcopolymerisaten aus Ethylenoxid oder Propylenoxid oder Copolymerisaten, die Ethylenoxid- und Propylenoxid-Gruppen eingebaut enthalten.
Geeignete Diole sind auch OH-terminierte Polyether-Homopolymere wie Polyethylenglykol, Polypropylenglykol und Polybutylenglykol, binäre Copolymere wie Ethylenglykol/Propylenglykol- und Ethylenglykol/Butylenglykol-Copolymere, geradkettige tertiäre Copolymere wie ternäre Ethylenglykol/Propylenglykol/Ethylenglykol-, Propylenglykol/Ethylenglykol/Propylenglykol- und Ethylenglykol/Butylenglykol/Ethylenglykol-Copolymere.
Geeignete Diole sind auch OH-terminierte Polyether-Blockcopolymere wie binäre Blockcopolymere wie Polyethylenglykol/Polypropylenglykol und Polyethylenglykol/ Polybutylenglycol, geradkettige, ternäre Blockcopolymere wie Polyethylenglykol/Polypropylenglykol/Polyethylenglykol, Polypropylenglykol/Polyethylenglycol/Polypropylenglykol und Polyethylenglykol/Polybutylengly- kol/Polyethylenglykol-Terpolymere.

Die vorgenannten Polyether können auch subsituiert sein und/oder von OH unterschiedliche Endgruppen aufweisen. Hierzu sei auf die DE 102 97 544, Absätze [0039] bis [0046] verwiesen, worauf hiermit in vollem Umfang Bezug genommen wird.

Besonders bevorzugte mehrwertige Alkohole sind solche mit 10 oder weniger Kohlenstoffatomen. Von diesen werden solche Alkohole bevorzugt, die bei 25°C und 1013 mbar im flüssigen Zustand vorliegen und eine so niedrige Viskosität aufweisen, dass sie ohne Zuhilfenahme einer weiteren flüssigen Phase als alleiniges Lösungs- und Dispergiermedium als Teil der Reaktionsmischung verwendet werden können. Beispiele solcher mehrwertigen Alkohole sind Ethylenglykol, Diethylenglykol, 1,2-Propandiol, 1,3-Propandiol, 1,2-Butandiol, 1,3-Butandiol, 1,4-Butandiol, 2,3-Butandiol, Pentandiol, Hexandiol und Octandiol, wobei Ethylenglykol (1,2-Ethandiol) und 1,2-Propandiol besonders bevorzugt sind.

Geeignete mehrwertige Alkohole sind auch Triole wie beispielsweise 1,1,1-Tris-(hydroxymethyl)ethan, 1,1,1-Tris-(hydroxymethyl)propan, 2-Ethyl-2-hydroxymethyl-1,3-propandiol, 1,2,6-Hexantriol, 1,2,3-Hexantriol und 1,2,4-Butantriol.

Als weitere mehrwertige Alkohole können auch Zuckeralkohole verwendet werden wie Glycerin, Threit, Erythrit, Pentaerythrit, Pentit, wobei der Pentit Xylit, Ribit und Arabit einschließt, Hexit, wobei der Hexit Mannit, Sorbit und Dulcit einschließt, Glycerinaldehyd, Dioxyaceton, Threose, Erythrulose, Erythrose, Arabinose, Ribose, Ribulose, Xylose, Xylulose, Lyxose, Glucose, Fructose, Mannose, Idose, Sorbose, Gulose, Talose, Tagatose, Galactose, Allose, Altrose, Lactose, Xylose, Arabinose, Isomaltose, Glucoheptose, Heptose, Maltotriose, Lactulose und Trehalose.

In einer weiteren Ausführungsform der Erfindung werden von den mehrwertigen Alkoholen Zuckeralkohole wie Glycerin, Threit, Erythrit, Pentaerythrit, Pentit und Hexit bevorzugt, da sie zu einer erhöhten Agglomerationsbeständigkeit der feinen Metalloxidteilchen in der Metalloxid-Dispersion führen.

Für geeignete Alkohole im Sinne dieser Erfindung sei weiterhin auf die Offenbarung der JP 2003268368 UEA1, S.11, Absatz [0026] hingewiesen, auf die hier in vollem Umfang Bezug genommen wird.

Die vorgenannten Alkohole können erfindungsgemäß allein oder in Mischungen davon verwendet werden.

Die Reaktionsmischung enthält nach Schritt a) des erfindungsgemäßen Verfahrens bevorzugt höchstens 99, besonders bevorzugt höchstens 95 und insbesondere höchstens 90 Gew.-% Alkohol, jeweils bezogen auf die Gesamtmasse der Reaktionsmischung.
Die Reaktionsmischung enthält nach Schritt a) des erfindungsgemäßen Verfahrens bevorzugt wenigstens 1, weiter bevorzugt wenigstens 10, besonders bevorzugt wenigstens 20 und ganz besonders bevorzugt wenigstens 30 Gew.-% Alkohol, jeweils bezogen auf die Gesamtmasse der Reaktionsmischung. Am meisten bevorzugt ist es, wenn die Reaktionsmischung nach Schritt a) des erfindungsgemäßen Verfahrens wenigstens 50 Gew.-% Alkohol enthält.

Die Reaktionsmischung kann außer der geeigneten Vorstufe und Alkohol wenigstens ein weiteres kosmetisch akzeptables organisches Lösungsmittel enthalten.

Geeignete organische Lösungsmittel sind beispielsweise flüssige Keton-Lösungsmittel, Amid-Lösungsmittel, Ester-Lösungsmittel und Ether-Lösungsmittel.

Die Keton-Lösungsmittel können beispielsweise ausgewählt werden unter Aceton, Methylethylketon, Methyl-n-propylketon, Methyl-n-butylketon, Diethylketon, Methylibutylketon, Methyl-n-pentylketon, Ethyl-n-butylketon, Methyl-n-hexylketon, Diibutylketon, Trimethylnonanon, Cyclohexanon, 2-Hexanon, Methylcyclohexanon, 2,4-Heptandion, Acetophenon, Acetylaceton, 2,4-Hexandion, 2,5-Hexandion, 2,4-Heptandion, 3,5-Heptandion, 2,4-Octandion, 3,5-Octandion, 2,4-Nonandion, 3,5-Nonandion, 5-Methyl-2,4-hexandion, 2,2,6,6-Tetramethyl-3,5-heptandion und 1,1,1, 5, 5, 5-Hexafluor- 2,4-heptandion.

Die Amid-Lösungsmittel können beispielsweise ausgewählt werden unter Formamid, N-Methylformamid, N,N-Dimethylformamid, N-Ethylformamid, N,N-Diethylformamid, Acetamid, N-Methylacetamid, N,N-Dimethylacetamid, N-Ethylacetamid, N,N-Diethylacetamid, N-Methylpropionamid, N-Methylpyrrolidon, N-Formylmorpholin, N-Formylpiperidin, N-Formylpyrrolidin, N-Acetylmorpholin, N-Acetylpiperidin und N-Acetylpyrrolidin.

Die Ester-Lösungsmittel können beispielsweise ausgewählt werden unter Diethylcarbonat, Ethylencarbonat, Propylencarbonat, Diethylcarbonat, Methylacetat, Ethylacetat, y-Butyrolacton, y-Valerolacton, n-Propylacetat, i-Propylacetat, n-Butylacetat, i-Butylacetat, sec-Butylacetat, n-Pentylacetat, sec-Pentylacetat, 3-Methoxybutylacetat, Methylpentylacetat, 2-Ethylbutylacetat, 2-Ethylhexylacetat, Benzylacetat, Cyclohexylacetat, Methylcyclohexylacetat, n-Nonylacetat, Methylacetoacetat, Ethylacetoacetat, Ethylenglykolmonomethyletheracetat, Ethylenglykolmonoethyletheracetat, Diethylenglykolmonomethyletheracetat, Diethylenglykolmonoethyletheracetat, Diethylenglykolmono-n-butyletheracetat, Propylenglykolmonomethyletheracetat, Propylenglykolmonoethyletheracetat, PropylenglykolmonopropyAetheracetat, Propylenglykolmonobutyletheracetat, Dipropylenglykolmonomethyletheracetat, Dipropylenglykolmonoethyletheracetat, Glykoldiacetat, Methoxytriglykolacetat, Ethylpropionat, n-Butylpropionat, i-Amylpropionat, Diethyloxalat, Di-n-butyloxalat, Methyllactat, Ethyllactat, n-Butyllactat, n-Amyllactat, Diethylmalonat, Dimethylphthalat und Diethylphthalat.

Die Ether-Lösungsmittel können beispielsweise ausgewählt werden unter Dipropylether, Diisopropylether, Dioxan, Tetrahydrofuran, Tetrahydropyran, Ethylenglykoldimethylether, Ethylenglykoldiethylether, Ethylenglykoldipropylether, Propylenglykoldimethylether, Propylenglykoldiethylether, Propylenglykoldipropylether, Diethylenglykoldimethylether, Diethylenglykoldiethylether und Diethylenglykoldipropylether.

Die vorgenannten Lösungsmittel können jeweils alleine oder als Mischungen davon verwendet werden.

### Herstellung des Metalloxids

Zur Herstellung des Metalloxids wird von einer geeigneten Vorstufe ausgegangen.

Das erfindungsgemäß besonders bevorzugte Metalloxid ist Zinkoxid. Geeignete Vorstufen für die Herstellung von Zinkoxid nach Schritt a) des erfindungsgemäßen Verfahrens sind Zinkcarboxylate. Dies sind im weitesten Sinne Zink-Verbindungen, die stöchiometrisch wenigstens eine Carboxylgruppe pro Zn-Atom besitzen. Dabei handelt es sich bevorzugt um partielle oder vollständige Zinksalze von gesättigten oder ungesättigten Monocarbonsäuren, gesättigten oder ungesättigten Polycarbonsäuren, alizyklischen oder aromatischen Mono- oder Polycarbonsäuren, wobei alle diese Säuren auch noch weitergehend substituiert sein können wie beispielsweise durch Hydroxy, Cyano, Halogen, Amino, Nitro, Alkoxy, Sulfon oder Halogen.
Besonders geeignete Säuren sind in der japanischen Offenlegungsschrift
JP 2003268368 UEA1, S. 11, Absatz [0025] genannt, worauf hiermit in vollem Umfang Bezug genommen wird.
Bevorzugt sind von diesen Zinkcarboxylaten solche, die außerdem Hydroxygruppen im Kristallgitter besitzen und durch die untenstehende allgemeine Formel I wiedergegeben werden.
Bevorzugt ist demnach das erfindungsgemäße Verfahren, dadurch gekennzeichnet, dass die in Schritt a) verwendete geeignete Vorstufe ausgewählt ist unter Verbindungen der allgemeinen Formel I

Zn(O)ₚ(OCOR)ₓ(OH)_{y}(OR')_{z} (I)

wobei Zn(O)ₚ(OCOR)ₓ(OH)_{y}(OR')_{z}
R bedeutet H, Alkyl, Cycloalkyl, Aryl, Arylalkyl
R' bedeutet Alkyl, Cycloalkyl, Aryl, Arylalkyl
p = (2-x-y-z)/2
x+y+z ≤ 2
0 < x ≤ 2
0 ≤ y < 2
0 ≤ z < 2

Unberücksichtigt in Formel I bleiben evtl. im Kristallgitter vorhandene Wasser- bzw. Lösungsmittelmoleküle. Erfindungsgemäß sind aber auch derartige Wasser- oder andere Lösungsmittel enthaltende Verbindungen von der allgemeinen Formel I umfasst.

Bevorzugt ist die in Schritt a) verwendete geeignete Vorstufe Zinkacetat-Dihydrat der Formel Zn(OCOCH₃)₂ * 2 H₂O.

Die Reaktionsmischung umfasst im Bereich von 1 bis 75, bevorzugt von 5 bis 50, besonders bevorzugt von 10 bis 25 und insbesondere von 10 bis 15 Gew.-% der geeigneten Vorstufe, bezogen auf das Gesamtgewicht aller für die Reaktion verwendeten Komponenten.

Die Reaktionsmischung umfasst im Bereich von 25 bis 99, bevorzugt von 50 bis 95, besonders bevorzugt von 75 bis 90 und insbesondere von 85 bis 90 Gew.-% Alkohol, bezogen auf das Gesamtgewicht aller für die Reaktion verwendeten Komponenten.

In einer weiteren Ausführungsform der Erfindung kann die Reaktionsmischung außer Alkohol und der geeigneten Vorstufe weitere Komponenten enthalten.

Wenn die geeignete Vorstufe kein Wasser enthält, beispielsweise in der Form von Kristallwasser, werden der Reaktionsmischung in einer bevorzugten Ausführungsform der Erfindung im Bereich von 0,5 bis 7,5 Gew.-% Wasser, bezogen auf das Gesamtgewicht aller für die Reaktion verwendeten Komponenten, zugegeben.

Das Gesamtgewicht aller für die Reaktion verwendeten Komponenten beträgt 100 Gew.-%.

Zur Durchführung von Schritt a) des erfindungsgemäßen Verfahrens wird zunächst die geeignete Vorstufe mit dem Alkohol in Kontakt gebracht.

Die Temperatur der die geeignete Vorstufe und Alkohol umfassenden Mischung beträgt wenigstens 50°C, bevorzugt wenigstens 70°C, besonders bevorzugt wenigstens 100°C und insbesondere wenigstens 150°C.

Die Temperatur der die geeignete Vorstufe und Alkohol umfassenden Mischung beträgt höchstens 300°C, bevorzugt höchstens 250°C, besonders bevorzugt wenigstens 220°C und insbesondere höchstens 200°C.

Erfindungsgemäß kann die Reaktionsmischung auf verschiedene Art und Weise auf die gewünschte Temperatur gebracht werden:
1) Gemeinsames Erwärmen der Mischung enthaltend die geeignete Vorstufe, den Alkohol und gegebenenfalls weitere Bestandteile;
2) Erwärmen des Alkohols und ggf. der übrigen Bestandteile der Reaktionsmischung und Hinzufügen der geeigneten Vorstufe;
3) Erwärmen der geeigneten Vorstufe und ggf. der übrigen Bestandteile der Reaktionsmischung und Hinzufügen des Alkohols;
4) Getrenntes Erwärmen aller Bestandteile der Mischung und anschließendes Mischen.

In einer bevorzugten Ausführungsform der Erfindung wird die Reaktionsmischung mit einer Heizrate r1 auf eine Temperatur T1 erwärmt, für eine bestimmte Zeit t1 bei dieser Temperatur T1 belassen und danach mit einer Heizrate r2 auf eine Temperatur T2, die größer ist als Temperatur T1 erwärmt und bei T2 wiederum für eine bestimmte Zeit t2 belassen.

Die Temperaturen T1 und T2 der die geeignete Vorstufe und Alkohol umfassenden Mischung betragen wenigstens 50°C, bevorzugt wenigstens 70°C, besonders bevorzugt wenigstens 100°C und insbesondere wenigstens 150°C, wobei T2 größer ist als T1.

Die Temperaturen T1 und T2 der die geeignete Vorstufe und Alkohol umfassenden Mischung betragen höchstens 300°C, bevorzugt höchstens 250°C, besonders bevorzugt wenigstens 220°C und insbesondere höchstens 200°C, wobei T2 größer ist als T1.

Eine bevorzugte Ausführungsform der Erfindung ist auch ein erfindungsgemäßes Verfahren, dadurch gekennzeichnet, dass die Reaktionsmischung in Schritt a) nacheinander auf zwei verschiedene Temperaturen T1 und T2 im Bereich von 50 bis 300°C, weiter bevorzugt im Bereich von 70 bis 200°C erwärmt wird, wobei T2 größer ist als T1.

Es ist bekannt, dass die Teilchengröße der Metalloxid-Partikel beispielsweise über die Heizraten r1 und r2 beeinflusst werden kann. Je nach gewünschter Teilchengröße muss die Heizrate entsprechend gewählt werden. Generell führen höhere Heizraten zu kleineren Teilchengrößen.

Die Reaktion kann mit oder ohne Kondensation der flüssigen Phase und deren Rückführung ("unter Rückfluss") durchgeführt werden.

In einer Ausführungsform der Erfindung wird die Reaktion für eine bestimmte Zeit t3 zunächst mit Kondensation der flüssigen Phase und deren Rückführung ("unter Rückfluss") und anschließend für eine bestimmte Zeit t4 nicht unter Rückfluss durchgeführt.

In einer bevorzugten Ausführungsform der Erfindung wird der wie vorgehend beschrieben erhaltenen Reaktionsmischung Wasser zugegeben. Diese zugegebene Wassermenge hängt von der Menge des bereits in der geeigneten Vorstufe beispielsweise als Kristallwasser vorhandenen Wassers ab, und soll zusammen mit dem in der Vorstufe bereits enthaltenen Wasser im Bereich von 0,1 bis 15 Gew.-%, bevorzugt von 0,5 bis 7,5 Gew.-%, besonders bevorzugt von 0,8 bis 3,5 Gew.-% liegen, jeweils bezogen auf die Gesamtmenge aller in der Reaktionsmischung enthaltenen Bestandteile.

In einer weiteren Ausführungsform der Erfindung wird der Reaktionsmischung eine organische Säure wie beispielsweise Essigsäure zugefügt
Schritt a) kann bei gegenüber der Umgebung gleichem, erhöhtem oder vermindertem Druck durchgeführt werden. Wenn die Temperatur der Reaktionsmischung den Siedepunkt der flüssigen Phase der Reaktionsmischung übersteigt, soll die Reaktion in drucksicheren Gefäßen durchgeführt werden.
Schritt b)
   Das erfindungsgemäße Verfahren kann Schritt b) enthalten. In Schritt b) werden bis zu 90 Gew.-% der flüchtigen Bestandteile der aus Schritt a) erhaltenen Metalloxid-Reaktionsmischung entfernt. Unter flüchtigen Bestandteilen werden hierbei insbesondere Alkohol und Lösungsmittel verstanden. Das Entfernen dieser Bestandteile erfolgt auf übliche, dem Fachmann bekannte Art und Weise.
   Beispielsweise erfolgt das Entfernen durch Verdampfen, Abdestillieren oder Zentrifugieren.
   Es ist ein Aspekt der Erfindung, dass das in Schritt a) hergestellte Metalloxid in Schritt b) weder in trockene Form noch in Pulverform überführt wird sondern mit einem Gehalt an Alkohol und gegebenenfalls weiteren Bestandteilen gegebenenfalls dem Schritt c) und danach dem Schritt d) zugeführt wird.
   In einer bevorzugten Ausführungsform der Erfindung werden im Bereich 25 bis 98 Gew.-%, besonders bevorzugt im Bereich von 50 bis 98 Gew.-% und insbesondere im Bereich von 85 bis 97 Gew.-% der flüchtigen Bestandteile, bezogen auf das Gesamtgewicht der Reaktionsmischung, entfernt.
   In einer anderen bevorzugten Ausführungsform der Erfindung werden Bestandteile, bevorzugt flüchtige Bestandteile der Reaktionsmischung derart entfernt, dass der Anteil von Metalloxiden im Bereich von 20 bis 75 Gew.-%, besonders bevorzugt im Bereich von 33 bis 66 Gew.-% und insbesondere im Bereich von 40 bis 60 Gew.-% liegt, bezogen auf das Gesamtgewicht der Reaktionsmischung nach der Entfernung der Bestandteile.
Schritt c)
   Das erfindungsgemäße Verfahren kann einen Schritt c) enthalten. In Schritt c) erfolgt ein wenigstens teilweises Austauschen der flüssigen Phase 1 der Reaktionsmischung gegen eine von der flüssigen Phase 1 unterschiedliche flüssige Phase 2. Dieser Austausch der flüssigen Phasen, gegebenenfalls auch als Lösungsmittelaustausch bezeichnet, erfolgt auf übliche, dem Fachmann bekannte Art und Weise, beispielsweise mittels eines Membranverfahrens wie Nano-, Ultra-, Mikro- oder Crossflowfiltration.
Schritt d)
   In Schritt d) des erfindungsgemäßen Verfahrens wird die nach den Schritten a), gegebenenfalls b) und gegebenenfalls c) erhaltene Reaktionsmischung unmittelbar, im Wesentlichen ohne weitere Aufarbeitung, als Grundlage für eine kosmetische Zubereitung verwendet oder einer bereits bestehenden kosmetischen Zubereitung hinzugefügt.

### Kosmetische Zubereitungen

Die nach dem erfindungsgemäßen Verfahren hergestellten kosmetischen Zubereitungen enthalten bevorzugt neben Metalloxid und Alkohol weiterhin wenigstens ein Antioxidationsmittel (Antioxidans).

Erfindungsgemäß können als Antioxidantien alle für kosmetische Anwendungen geeigneten oder gebräuchlichen Antioxidantien verwendet werden. Vorteilhaft werden die Antioxidantien gewählt aus der Gruppe bestehend aus Aminosäuren (z.B. Glycin, Histidin, Tyrosin, Tryptophan) und deren Derivaten, Imidazolen (z.B. Urocaninsäure) und deren Derivaten, Peptiden wie D,L-Carnosin, D-Carnosin, L-Carnosin und deren Derivaten (z.B. Anserin), Carotinoide, Carotinen (z.B. α-Carotin, β-Carotin, γ-Lycopin) und deren Derivaten, Chlorogensäure und deren Derivaten, Liponsäure und deren Derivaten (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Coniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol , Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zusammensetzungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zusammensetzung.

Sofern Vitamin E und/oder dessen Derivate das oder die Antioxidantien darstellen, ist es vorteilhaft, diese in Konzentrationen von 0,001 bis 10 Gew.-%, bezogen auf das Gesamtgewicht der Zusammensetzung, bereitzustellen.

Sofern Vitamin A, bzw. Vitamin-A-Derivate, bzw. Carotine bzw. deren Derivate das oder die Antioxidantien darstellen, ist vorteilhaft, diese in Konzentrationen von 0,001 bis 10 Gew.-%, bezogen auf das Gesamtgewicht der Zusammensetzung, bereitzustellen.

Die nach dem erfindungsgemäßen Verfahren hergestellten kosmetischen Zubereitungen weisen bevorzugt neben Metalloxid und Alkohol weiterhin wenigstens eine kosmetisch akzeptable Öl- bzw. Fettkomponente auf, die ausgewählt ist unter: Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestern; Vaselin; Estern, bevorzugt Estern von Fettsäuren, wie z. B. die Ester von C₁-C₂₄-Monoalkoholen mit C₁-C₂₂-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexancosanylstearat, Octacosanylstearat, Triacontanylstearat, Dotriacontanylstearat, Tetratriacontanylstearat; Salicylaten, wie C₁-C₁₀-Salicylaten, z. B. Octylsalicylat; Benzoatestern, wie C₁₀-C₁₅-Alkylbenzoaten, Benzylbenzoat; anderen kosmetischen Estern, wie Fettsäuretriglyceriden, Propylenglykolmonolaurat, Polyethylenglykolmonolaurat, C₁₀-C₁₅-Alkyllactaten, etc. und Mischungen davon.

Geeignete Siliconöle sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinus-öl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete kosmetisch und pharmazeutisch verträgliche Öl- bzw. Fettkomponenten sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird. Vorteilhafterweise werden diejenigen Öle, Fette und/oder Wachse gewählt, die auf Seite 28, Zeile 39 bis Seite 34, Zeile 22 der WO 2006/106140 beschrieben sind. Auf den Inhalt der genannten Textstelle wird hiermit in vollem Umfang Bezug genommen.
Der Gehalt an weiteren Ölen, Fetten und Wachsen beträgt höchstens 50, bevorzugt 30, weiter bevorzugt höchstens 20 Gewichts-%, bezogen auf das Gesamtgewicht der Zubereitung.

Geeignete hydrophile Träger sind ausgewählt unter Wasser, ein-, zwei- oder mehrwertigen Alkoholen mit vorzugsweise 1 bis 8 Kohlenstoffatomen, wie Ethanol, n-Propanol, iso-Propanol, Propylenglycol, Glycerin, Sorbit, etc.

Bei den kosmetischen Zubereitungen kann es sich um hautkosmetische, haarkosmetische, dermatologische, hygienische oder pharmazeutische Zubereitungen handeln.

Vorzugsweise liegen die Zubereitungen in Form eines Gels, Schaums, Sprays, einer Salbe, Creme, Emulsion, Suspension, Lotion, Milch oder Paste vor. Gewünschtenfalls können auch Liposomen oder Mikrosphären eingesetzt werden.

Die kosmetischen Zubereitungen enthalten bevorzugt außer Metalloxid und Alkohol zusätzliche kosmetisch und/oder dermatologisch aktive Wirkstoffe sowie Hilfsstoffe.

Vorzugsweise enthalten die kosmetischen Zubereitungen wenigstens einen weiteren Bestandteil, der ausgewählt ist unter kosmetisch aktiven Wirkstoffen, Emulgatoren, Tensiden, Konservierungsmitteln, Parfümölen, Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweisshydrolysaten, Lipiden, Antioxidantien, Entschäumern, Antistatika, Emollienzien und Weichmachern.

Die kosmetischen Zubereitungen können als wässrige oder wässrig-alkoholische Lösungen, O/W- sowie W/O-Emulsionen, Hydrodispersionsformulierungen, feststoffstabilisierte Formulierungen, Stiftformulierungen, PIT-Formulierungen, in Form von Cremes, Schäumen, Sprays (Pumpspray oder Aerosol), Gelen, Gelsprays, Lotionen, Ölen, ölgelen oder Mousse vorliegen und dementsprechend mit üblichen weiteren Hilfsstoffen formuliert werden.

Kosmetische Zubereitungen im Sinne der Erfindung sind auch Haarpflegemittel ausgewählt aus der Gruppe bestehend aus Vorbehandlungsmitteln, Haarspülungen, Haarconditionern, Haarbalsamen, leave-on-Haarkuren, rinse-off-Haarkuren, Haarwässern, Pomaden, Frisiercremes, Frisierlotionen, Frisiergelen, Spitzenfluids, Hot-Oil-Treatments und Schaumkuren.

Weiterhin betrifft die Erfindung die Herstellung von kosmetischen Zubereitungen, die ausgewählt sind aus Gelcremes, Hydroformulierungen, Stiftformulierungen, kosmetischen Ölen und Ölgelen, Mascara, Selbstbräunern, Gesichtspflegemitteln, Körperpflegemitteln, After-Sun-Präparaten, Haarverformungsmitteln und Haarfestigern.

Weitere kosmetische Zubereitungen sind hautkosmetische Zubereitungen, insbesondere solche zur Pflege der Haut. Diese liegen insbesondere als W/O- oder O/W-Hautcremes, Tag- und Nachtcremes, Augencremes, Gesichtscremes, Antifaltencremes, Mimikcremes, Feuchthaltecremes, Bleichcremes, Vitamincremes, Hautlotionen, Pflegelotionen und Feuchthaltelotionen vor.
Weitere kosmetische Zubereitungen sind Gesichtswässer, Gesichtsmasken, Deodorantien und andere kosmetische Lotionen und Zubereitungen für die Verwendung in der dekorativen Kosmetik, beispielsweise als Abdeckstift, Theaterfarbe, in Mascara und Lidschatten, Lippenstiften, Kajalstiften, Eyelinern, Makeup, Grundierungen, Rouges und Pudern und Augenbrauenstiften.

Außerdem können die erfindungsgemäß hergestellten Zubereitungen verwendet werden in Nose-Strips zur Porenreinigung, in Antiaknemitteln, Repellentien, Rasiermitteln, Haarentfernungsmitteln, Intimpflegemitteln, Fußpflegemitteln sowie in der Babypflege.

Weitere nach dem erfindungsgemäßen Verfahren erhältliche kosmetische Zubereitungen sind Wasch-, Dusch- und Badepräparate.
Unter Wasch-, Dusch- und Badepräparaten werden im Rahmen dieser Erfindung Seifen von flüssiger bis gelförmiger Konsistenz, wie Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und -cremes verstanden. Geeignete weitere Inhaltsstoffe für diese erfindungsgemäß hergestellten Wasch-, Dusch- und Badepräparate sind im Folgenden beschrieben.

Die kosmetischen Zubereitungen enthalten bevorzugt weitere kosmetisch akzeptable Zusätze wie beispielsweise Emulgatoren und Co-Emulgatoren, Lösungsmittel, Tenside, Ölkörper, Konservierungsmittel, Parfümöle, kosmetische Pflege- und Wirkstoffe wie AHA-Säuren, Fruchtsäuren, Ceramide, Phytantriol, Collagen, Vitamine und Provitamine, beispielsweise Vitamin A, E und C, Retinol, Bisabolol, Panthenol, natürliche und synthetische Lichtschutzmittel, Naturstoffe, Trübungsmittel, Lösungsvermittler, Repellentien, Bleichmittel, Färbemittel, Tönungsmittel, (Selbst-)Bräunungsmittel (z.B. Dihydroxyaceton, Tyrosin, Canthaxanthin, Melanotan), weitere Mikropigmente wie Titandioxid, Überfettungsmittel, Perlglanzwachse, Konsistenzgeber, Verdicker, Solubilisatoren, Komplexbildner, Fette, Wachse, Silikonverbindungen, Hydrotrope, Farbstoffe, Stabilisatoren, pH-Wert Regulatoren, Reflektoren, Proteine und Proteinhydrolysate (z.B. Weizen-, Mandel- oder Erbsenproteine), Ceramid, Eiweißhydrolysate, Salze, Gelbildner, Konsistenzgeber, Silikone, Feuchthaltemittel (z.B. 1,2-Pentandiol), Rückfetter, UV-Lichtschutzfilter und weitere übliche Additive. Des weiteren können zur Einstellung der jeweils gewünschten Eigenschaften insbesondere auch weitere Polymere enthalten sein.

Die kosmetischen Zubereitungen enthalten bevorzugt wenigstens ein Selbstbräunungsmittel.
Die kosmetischen Zubereitungen enthalten bevorzugt wenigstens einen weiteren Alkohol und/oder wenigstens ein Öl. Bevorzugt wird die Menge von Alkohol und/oder Öl so gewählt, dass gewünschte Wirkstoffe wie beispielsweise organische UV-Filter dadurch in den gelösten Zustand überführt werden.
Die kosmetischen Zubereitungen enthalten bevorzugt wenigstens einen weiteren anorganischen UV-Lichtschutzfilter.
Die kosmetischen Zubereitungen enthalten bevorzugt wenigstens einen Acrylsäure-Verdicker.
Die kosmetischen Zubereitungen können auch Tenside enthalten.

### Tenside

Als Tenside können anionische, kationische, nichtionische und/oder amphotere Tenside eingesetzt werden.
Vorteilhafte waschaktive anionische Tenside im Sinne der vorliegenden Erfindung sind - Acylaminosäuren und deren Salze, wie Acylglutamate, insbesondere Natriumacylglutamat
- Sarcosinate, beispielsweise Myristoyl Sarcosin, TEA-Lauroyl Sarcosinat, Natriumlauroylsarcosinat und Natriumcocoylsarcosinat,
   Sulfonsäuren und deren Salze, wie
- Acylisethionate, beispielsweise Natrium- oder Ammoniumcocoylisethionat
- Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat, Dinatrium PEG-5 Laurylcitratsulfosuccinat und Derivate,
- Alkylethersulfate, beispielsweise Natrium-, Ammonium-, Magnesium-, MIPA-, TIPA-Laurethsulfat, Natriummyrethsulfat und Natrium C₁₂₋₁₃ Parethsulfat,
- Alkylsulfate, beispielsweise Natrium-, Ammonium- und TEA-Laurylsulfat. Weitere vorteilhafte anionische Tenside sind
- Taurate, beispielsweise Natriumlauroyltaurat und Natriummethylcocoyltaurat,
- Ether-Carbonsäuren, beispielsweise Natriumlaureth-13 Carboxylat und Natrium PEG-6 Cocamide Carboxylat, Natrium PEG-7-Olivenöl-Carboxylat
- Phosphorsäureester und Salze, wie beispielsweise DEA-Oleth-10 Phosphat und Dilaureth-4 Phosphat,
- Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat,
- Acylglutamate wie Di-TEA-palmitoylaspartat und Natrium Caprylic/Capric Glutamat,
- Acylpeptide, beispielsweise Palmitoyl hydrolysiertes Milchprotein, Natrium Cocoyl hydrolysiertes Sojaprotein und Natrium-/Kalium Cocoyl hydrolysiertes Kollagen sowie Carbonsäuren und Derivate, wie beispielsweise Laurinsäure, Aluminiumstearat, Magnesiumalkanolat und Zinkundecylenat, Ester-Carbonsäuren, beispielsweise Calciumstearoyllactylat, Laureth-6 Citrat und Natrium PEG-4 Lauramidcarboxylat
- Alkylarylsulfonate.

Vorteilhafte waschaktive kationische Tenside im Sinne der vorliegenden Erfindung sind quaternäre Tenside. Quaternäre Tenside enthalten mindestens ein N-Atom, das mit 4 Alkyl- oder Arylgruppen kovalent verbunden ist. Vorteilhaft sind beispielsweise Alkylbetain, Alkylamidopropylbetain und Alkylamidopropylhydroxysultain.
Weitere vorteilhafte kationische Tenside im Sinne der vorliegenden Erfindung sind ferner
- Alkylamine,
- Alkylimidazole und
- ethoxylierte Amine
und insbesondere deren Salze.
Vorteilhafte waschaktive amphotere Tenside im Sinne der vorliegenden Erfindung sind Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat, Natriumacylamphopropionat, und N-Kokosfettsäureamidoethyl-N-hydroxyethylglycinat Natriumsalze.
Weitere vorteilhafte amphotere Tenside sind N-Alkylaminosäuren, beispielsweise Aminopropylalkylglutamid, Alkylaminopropionsäure, Natriumalkylimidodipropionat und Lauroamphocarboxyglycinat.
Vorteilhafte waschaktive nicht-ionische Tenside im Sinne der vorliegenden Erfindung sind
- Alkanolamide, wie Cocamide MEA/DEA/MIPA,
- Ester, die durch Veresterung von Carbonsäuren mit Ethylenoxid, Glycerin, Sorbitan oder anderen Alkoholen entstehen,
- Ether, beispielsweise ethoxylierte Alkohole, ethoxyliertes Lanolin, ethoxylierte Polysiloxane, propoxylierte POE Ether, Alkylpolyglycoside wie Laurylglucosid, Decylglycosid und Cocoglycosid, Glycoside mit einem HLB-Wert von wenigstens 20 (z.B. Belsil^{®}SPG 128V (Wacker)).
Weitere vorteilhafte nicht-ionische Tenside sind Alkohole und Aminoxide, wie Cocoamidopropylamin-Oxid.

Bevorzugte anionische, amphotere und nichtionische Shampoo-Tenside sind beispielsweise in "Kosmetik und Hygiene von Kopf bis Fuß", Hrsg. W. Umbach, 3. Auflage, Wiley-VCH, 2004, S.131-134 genannt, worauf an dieser Stelle in vollem Umfang Bezug genommen wird.
Unter den Alkylethersulfaten sind insbesondere Natriumalkylethersulfate auf Basis von zwei- oder dreifach ethoxyliertem Lauryl- und Myristylalkohol bevorzugt. Sie übertreffen die Alkylsulfate deutlich bezüglich der Unempfindlichkeit gegen Wasserhärte, der Verdickbarkeit, der Kältelöslichkeit und insbesondere der Haut- und Schleimhautverträglichkeit. Sie können auch als alleinige Waschrohstoffe für Shampoos eingesetzt werden können. Laurylethersulfat weist bessere Schaumeigenschaften als Myristylethersulfat auf, ist diesem aber in der Milde unterlegen.
Alkylethercarboxylate mit mittlerem und besonders mit höherem gehören zu den mildesten Tensiden überhaupt, zeigen aber ein schlechtes Schaum- und Viskositätsverhalten. Sie werden oft in Kombination mit Alkylethersulfaten und amphoteren Tensiden in Haarwaschmitteln eingesetzt.
Sulfobemsteinsäureester (Sulfosuccinate) sind mild und gut schäumende Tenside werden aber wegen ihrer schlechten Verdickbarkeit bevorzugt nur zusammen mit anderen anionischen und amphoteren Tensiden und wegen ihrer geringen Hydrolysestabilität bevorzugt nur in neutralen bzw. gut gepufferten Produkten eingesetzt. Arnidopropylbetaine sind als alleinige Waschrohstoffe praktisch unbedeutend, da ihr Schaumverhalten sowie ihre Verdickbarkeit nur mäßig ausgeprägt sind. Demgegenüber weisen diese Tenside eine ausgezeichnete Haut- und Augenschleimhautverträglichkeit auf. In Kombination mit anionischen Tensiden lässt sich deren Milde synergistisch verbessern. Bevorzugt ist die Verwendung von Cocamidopropylbetain. Arnphoacetate/Arnphodiacetate besitzen als amphotere Tenside eine sehr gute Haut- und Schleimhautverträglichkeit und können haarkonditionierend wirken bzw. die Pflegewirkung von Zusatzstoffen erhöhen. Sie werden ähnlich wie die Betaine zur Optimierung von Alkylethersulfat-Formulierungen eingesetzt. Am meisten bevorzugt sind Natriumcocoamphoacetat und Dinatriumcocoamphodiacetat.

Alkylpolyglykoside sind nichtionische Waschrohstoffe. Sie sind mild, haben gute Universaleigenschaften, schäumen jedoch schwach. Aus diesem Grund werden sie bevorzugt in Kombinationen mit anionischen Tensiden verwendet.
Sorbitanester gehören ebenfalls zu den nichtionischen Waschrohstoffen. Wegen ihrer ausgezeichneten Milde werden sie bevorzugt zum Einsatz bei Baby-Shampoos verwendet. Als Schwachschäumer werden sie bevorzugt in Kombination mit anionischen Tensiden eingesetzt.
Es ist vorteilhaft, das oder die waschaktiven Tenside aus der Gruppe der Tenside zu wählen, welche einen HLB-Wert von mehr als 25 haben, besonders vorteilhaft sind solche, welchen einen HLB-Wert von mehr als 35 haben.
Es ist erfindungsgemäß von Vorteil, wenn ein oder mehrere dieser Tenside in einer Konzentration von 1 bis 30 Gewichts-%, bevorzugt in einer Konzentration 5 von 25 bis Gewichts-% und ganz besonders bevorzugt in einer Konzentration von 10 bis 20 Gewichts-%, jeweils bezogen auf das Gesamtgewicht der Zubereitung eingesetzt werden.

### Polysorbate

Als waschaktive Agentien können auch Polysorbate vorteilhaft in die kosmetischen Zubereitungen eingearbeitet werden.
Im Sinne der Erfindung vorteilhafte Polysorbate sind beispielsweise
- Polyoxyethylen(20)sorbitanmonolaurat (Tween^{®}20, CAS-Nr. 9005-64-5)
- Polyoxyethylen(4)sorbitanmonolaurat (Tween^{®}21, CAS-Nr. 9005-64-5)
- Polyoxyethylen(4)sorbitanmonostearat (Tween^{®}61, CAS-Nr. 9005-67-8)
- Polyoxyethylen(20)sorbitantristearat (Tween^{®}65, CAS-Nr. 9005-71 -4)
- Polyoxyethylen(20)sorbitanmonooleat (Tween^{®}80, CAS-Nr. 9005-65-6)
- Polyoxyethylen(5)sorbitanmonooleat (Tween^{®}81, CAS-Nr. 9005-65-5)
- Polyoxyethylen(20)sorbitantrioleat (Tween^{®}85, CAS-Nr. 9005-70-3).
Besonders vorteilhaft sind
- Polyoxyethylen(20)sorbitanmonopalmitat (Tween^{®}40, CAS-Nr. 9005-66-7) und
- Polyoxyethylen(20)sorbitanmonostearat (Tween^{®}60, CAS-Nr. 9005-67-8).
Die Polysorbate werden vorteilhaft in einer Konzentration von 0,1 bis 5 und insbesondere in einer Konzentration von 1,5 bis 2,5 Gew.-%, bezogen auf das Gesamtgewicht der Zubereitung einzeln oder als Mischung mehrer Polysorbate, eingesetzt.

### Konditionierungsmittel

Sofern gewünscht, können die kosmetischen Zubereitungen auch Konditionierungsmittel enthalten. Bevorzugt werden dann die Konditionierungsmittel gewählt, die auf Seite 34, Zeile 24 bis Seite 37, Zeile 10 der WO 2006/106140 beschrieben sind. Auf den Inhalt der genannten Textstelle wird hiermit in vollem Umfang Bezug genommen.

### Rheologiemodifizierungsmittel

Bei den geeigneten Rheologiemodifizierungsmittein handelt es sich vor allem um Verdicker. Für Shampoos und Haarpflegemittel geeignete Verdicker sind in "Kosmetik und Hygiene von Kopf bis Fuß", Hrsg. W. Umbach, 3. Auflage, Wiley-VCH, 2004, S.235-236 genannt, worauf an dieser Stelle vollumfänglich Bezug genommen wird.
Geeignete Verdickungsmittel für die kosmetischen Zubereitungen sind beispielsweise auch auf Seite 37, Zeile 12 bis Seite 38, Zeile 8 der WO 2006/106140 beschrieben. Auf den Inhalt der genannten Textstelle wird hiermit in vollem Umfang Bezug genommen. Die kosmetischen Zubereitungen enthalten bevorzugt wenigstens einen Acrylsäure-Verdicker (INCI: Carbomer).

### Konservierungsmittel

Die kosmetischen Zubereitungen können auch Konservierungsmittel enthalten. Zubereitungen mit hohen Wassergehalten müssen zuverlässig vor Verkeimung geschützt sein. Geeignete Konservierungsmittel für die erfindungsgemäß hergestellten kosmetischen Zubereitungen sind beispielsweise auf Seite 38, Zeile 10 bis Seite 39, Zeile 18 der WO 2006/106140 beschrieben. Auf den Inhalt der genannten Textstelle wird hiermit in vollem Umfang Bezug genommen.

Komplexbildner: Da die Rohstoffe und auch die Shampoos selbst überwiegend in Stahlapparaturen hergestellt werden, können die Endprodukte Eisen(-Ionen) in Spurenmengen enthalten. Um zu verhindern, dass diese Verunreinigungen über Reaktionen mit Farbstoffen und Parfümölbestandteilen die Produktqualität nachteilig beeinflussen, werden Komplexbildner wie Salze der Ethylendiamintetraessigsäure, der Nitrilotriessigsäure, der Iminodibernsteinsäure oder Phosphate zugesetzt.

UV- Lichtschutzfilter: Um die in den kosmetischen Zubereitungen enthaltenen Inhaltsstoffe wie beispielsweise Farbstoffe und Parfümöle gegen Veränderungen durch UV-Licht zu stabilisieren, können UV- Lichtschutzfilter, wie z. B. Benzophenon-Derivate, eingearbeitet werden. Geeignete UV- Lichtschutzfilter für die erfindungsgemäß hergestellten kosmetischen Zubereitungen sind beispielsweise auf Seite 39, Zeile 20 bis Seite 41 Zeile 10 der WO 2006/106140 beschrieben. Auf den Inhalt der genannten Textstelle wird hiermit in vollem Umfang Bezug genommen.

Puffer: Puffer gewährleisten die pH-Wert-Stabilität der kosmetischen Zubereitungen. Überwiegend verwendet werden Citrat-, Lactat- und Phosphat-Puffer.

Lösungsvermittler: Sie werden eingesetzt, um pflegende Öle oder Parfümöle klar in Lösung zu bringen und auch in der Kälte klar in Lösung zu halten. Die gängigsten Lösungsvermittler sind ethoxylierte nichtionische Tenside, z. B. hydrierte und ethoxylierte Ricinusöle.

Keimhemmende Mittel: Weiterhin können auch keimhemmende Mittel eingesetzt werden. Dazu gehören generell alle geeigneten Konservierungsmittel mit spezifischer Wirkung gegen grampositive Bakterien, z.B. Triclosan (2,4,4'-Trichlor-2'-hydroxydiphenylether), Chlorhexidin (1,1'-Hexamethylenbis[5-(4-chlorphenyl)-biguanid) sowie TTC (3,4,4'-Trichlorcarbanilid). Quartäre Ammonium-Verbindungen sind prinzipiell ebenfalls geeignet und werden bevorzugt für desinfizierende Seifen und Waschlotionen verwendet. Auch zahlreiche Riechstoffe haben antimikrobielle Eigenschaften. Auch eine große Anzahl etherischer Öle bzw. deren charakteristische Inhaltsstoffe wie z.B. Nelkenöl (Eugenol), Minzöl (Menthol) oder Thymianöl (Thymol), zeigen eine ausgeprägte antimikrobielle Wirksamkeit.
Die antibakteriell wirksamen Stoffe werden in der Regel in Konzentrationen von ca. 0,1 bis 0,3 Gew.-% eingesetzt.

Dispergiermittel: Wenn in den kosmetischen Zubereitungen unlösliche Wirkstoffe, z.B. Antischuppenwirkstoffe oder Siliconöle, dispergiert und auf Dauer in Schwebe gehalten werden sollen, müssen Dispergiermittel und Verdicker wie z. B. Magnesium-Aluminium-Silicate, Bentonite, Fettacyl-Derivate, Polyvinylpyrrolidon oder Hydrokolloide, z. B. Xanthan Gum oder Carbomere, eingesetzt werden.
Erfindungsgemäß sind Konservierungsmittel in einer Gesamtkonzentration von höchstens 2, bevorzugt höchstens 1,5 und besonders bevorzugt höchstens 1 Gewichts-%, bezogen auf das Gesamtgewicht der Zubereitung, enthalten.

Die kosmetischen Zubereitungen können außer den vorgenannten Substanzen gegebenenfalls weitere, in der Kosmetik übliche Zusatzstoffe, beispielsweise Parfüm, Farbstoffe, rückfettende Agentien, Komplexierungs- und Sequestrierungsagentien, Perlglanzagentien, Pflanzenextrakte, Vitamine, Wirkstoffe, Pigmente, die eine färbende Wirkung haben, weichmachende, anfeuchtende und/oder feuchthaltende Substanzen, oder andere übliche Bestandteile einer kosmetischen oder dermatologischen Formulierung wie Alkohole, Polyole, Polymere, organische Säuren zur pH-Wert-Einstellung, Schaumstabilisatoren, Elektrolyte, organische Lösemittel oder Silikonderivate enthalten. Hinsichtlich der genannten, dem Fachmann bekannten weiteren Inhaltsstoffe für die Zubereitungen sei auf "Kosmetik und Hygiene von Kopf bis Fuß", Hrsg. W. Umbach, 3. Auflage, Wiley-VCH, 2004, S.123-128 verwiesen, worauf an dieser Stelle vollumfänglich Bezug genommen wird.

### Ethoxylierte Glycerin-Fettsäureester

Die kosmetischen Zubereitungen enthalten gegebenenfalls ethoxylierte Öle ausgewählt aus der Gruppe der ethoxylierten Glycerin-Fettsäureester, insbesondere bevorzugt PEG-10 Olivenölglyceride, PEG-11 Avocadoölglyceride, PEG-11 Kakaobutterglyceride, PEG-13 Sonnenblumenölglyceride, PEG-15 Glycerylisostearat, PEG-9 Kokosfettsäureglyceride, PEG-54 Hydriertes Ricinusöl, PEG-7 Hydriertes Ricinusöl, PEG-60 Hydriertes Ricinusöl, Jojobaöl Ethoxylat (PEG-26 Jojoba-Fett-Säuren, PEG-26 Jojobaalkohol), Glycereth-5 Cocoat, PEG-9 Kokosfettsäureglyceride, PEG-7 Glycerylcocoat, PEG-45 Palmkemölglyceride, PEG-35 Ricinusöl, Olivenöl-PEG-7 Ester, PEG-6 Caprylisäure/Caprinsäureglyceride, PEG-10 Olivenölglyceride, PEG-13 Sonnenblumenölglyceride, PEG-7 Hydriertes Ricinusöl, Hydrierte Palmkernölglycerid-PEG-6 Ester, PEG-20 Maisölglyceride, PEG- 18 Glycerylolead-cocoat, PEG-40 Hydriertes Ricinusöl, PEG-40 Ricinusöl, PEG-60 Hydriertes Ricinusöl, PEG-60 Maisölglyceride, PEG-54 Hydriertes Ricinusöl, PEG-45 Palmkernölglyceride, PEG-80 Glycerylcocoat, PEG-60 Mandelölglyceride, PEG-60 "Evening Primrose" Glyceride, PEG-200 Hydriertes Glycerylpalmat, PEG-90 Glycerylisostearat.
Bevorzugte ethoxylierte Öle sind PEG-7 Glycerylcocoat, PEG-9 Kokosglyceride, PEG-40 Hydriertes Rizinusöl, PEG-200 hydriertes Glycerylpalmat.
Ethoxylierte Glycerin-Fettsäureester werden in wässrigen Reinigungsrezepturen zu verschiedenen Zwecken eingesetzt. Glycerin-Fettsäureester mit einem Ethoxylierungsgrad von ca. 30-50 dienen als Lösungsvermittler für unpolare Substanzen wie Parfumöle. Hochethoxylierte Glycerin-Fettsäureester werden als Verdicker eingesetzt.

### Wirkstoffe

In die kosmetischen Zubereitungen lassen sich verschiedenste Wirkstoffe mit unterschiedlicher Löslichkeit homogen einarbeiten. Vorteilhafte Wirkstoffe in den kosmetischen Zubereitungen sind beispielsweise auf Seite 44, Zeile 24 bis Seite 49 Zeile 39 der WO 2006/106140 beschrieben. Auf den Inhalt der genannten Textstelle wird hiermit in vollem Umfang Bezug genommen.

### UV-Lichtschutzfilter

Die nach dem erfindungsgemäßen Verfahren erhätlichen kosmetischen Zubereitungen enthalten in einer bevozugten Ausführungsform wenigstens einen organischen UV-Lichtschutzfilter. Solche organischen UV-Lichtschutzfilter sind beispielsweise:

| Nr. | Stoff | CAS-Nr. (=Säure) |
|---|---|---|
| 1 | 4-Aminobenzoesäure | 150-13-0 |
| 2 | 3-(4'-Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfonsäure-(Sulisobenzonum) und das Natriumsalz | 4065-45-6 |
| 13 | 3-(4'-Sulfobenzyliden)-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 3-Imidazol-4-yl-acrylsäure und ihr Ethylester | 104-98-3 |
| 18 | 2-Cyano-3,3-diphenylacrylsäureethylester | 5232-99-5 |
| 19 | 2-Cyano-3,3-diphenylacrylsäure-2'-ethylhexylester | 6197-30-4 |
| 20 | Menthyl-o-aminobenzoat oder: 5-Methyl-2-(1-methylethyl)-2-am i nobenzoat | 134-09-8 |
| 21 | Glyceryl p-aminobenzoat oder: 4-Aminobenzoesäure-1-glyceryl-ester | 136-44-7 |
| 22 | 2,2'-Dihydroxy-4-methoxybenzophenon (Dioxybenzone) | 131-53-3 |
| 23 | 2-Hydroxy-4-methoxy-4-methylbenzophenon (Mexenon) | 1641-17-4 |
| 24 | Triethanolamin Salicylat | 2174-16-5 |
| 25 | Dimethoxyphenylglyoxalsäure oder: 3,4-dimethoxy-phenyl-glyoxal-saures Natrium | 4732-70-1 |
| 26 | 3-(4'Sulfobenzyliden)-bornan-2-on und seine Salze | 56039-58-8 |
| 27 | 4-tert.-Butyl-4'-methoxy-dibenzoylmethan | 70356-09-1 |
| 28 | 2,2',4,4'-Tetrahydroxybenzophenon | 131-55-5 |
| 29 | 2,2'-Methylen-bis-[6-(2H-benzotriazol-2-yl)-4-(1,1,3,3,-tetramethylbutyl)phenol] | 103597-45-1 |
| 30 | 2,2'-(1,4-Phenylen)-bis-1H-benzimidazol-4,6-disulfonsäure, Na-Salz | 180898-37-7 |
| 31 | 2,4-bis-[4-(2-Ethylhexyloxy)-2-hydroxy]phenyl-6-(4-methoxyphenyl)-(1,3,5)-triazin | 187393-00-6 |
| 32 | 3-(4-Methylbenzyliden)-campher | 36861-47-9 |
| 33 | 4-Bis(polyethoxy)paraaminobenzoesäurepolyethoxyethylester | 113010-52-9 |
| 34 | 2,4-Dihydroxybenzophenon | 131-56-6 |
| 35 | 2,2'-Dihydroxy-4,4'-dimethoxybenzophenon-5,5'-dinatriumsulfonat | 3121-60-6 |
| 36 | Benzoesäure, 2-[4-(diethylamino)-2-hydroxybenzoyl]-hexylester | 302776-68-7 |
| 37 | 2-(2H-Benzotriazol-2-yl)-4-methyl-6-[2-methyl-3-[1,3,3,3-tetramethyl-1-[(trimethylsilyl)oxy]disiloxanyl]propyl]phenol | 155633-54-8 |
| 38 | 1,1-[(2,2'-Dimethylpropoxy)carbonyl]-4,4-diphenyl-1,3-butadien | 363602-15-7 |

Auch polymere oder polymergebundene Filtersubstanzen können erfindungsgemäß verwendet werden.

Die erfindungsgemäß hergestellten kosmetischen Zubereitungen können vorteilhafterweise außerdem weitere anorganische Pigmente auf Basis von Metalloxiden und/oder anderen in Wasser schwerlöslichen oder unlöslichen Metallverbindungen, ausgewählt aus der Gruppe der Oxide des Titans (z.B. TiO₂), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden enthalten. Die anorganischen Pigmente können dabei in gecoateter Form vorliegen, d.h. dass sie oberflächlich behandelt sind. Diese Oberflächenbehandlung kann beispielsweise darin bestehen, dass die Pigmente nach an sich bekannter Weise, wie in DE-A-33 14 742 beschrieben, mit einer dünnen hydrophoben Schicht versehen sind. Zur Verwendung in den erfindungsgemäß hergestellten kosmetischen Zubereitungen geeignete Lichtschutzmittel sind die in der EP-A 1 084 696 in den Absätzen [0036] bis [0053] genannten Verbindungen, worauf an dieser Stelle vollumfänglich Bezug genommen wird. Für die erfindungsgemäße Verwendung geeignet sind alle UV-Lichtschutzfilter, die in Anlage 7 (zu § 3b) der deutschen Kosmetik-Verordnung unter "Ultraviolett-Filter für kosmetische Mittel" genannt sind.

Die Aufzählung der genannten UV-Lichtschutz-Filter, die in den erfindungsgemäßen Zusammensetzungen verwendet werden können, ist nicht abschließend.
Vorteilhaft enthalten die Zubereitungen Substanzen, die UV-Strahlung im UVB-Bereich und Substanzen, die UV-Strahlung im UVA-Bereich absorbieren, wobei die Gesamtmenge der Filtersubstanzen z.B. 0,1 bis 30 Gew.-%, vorzugsweise 0,5 bis 20 Gew.-%, insbesondere 1 bis 15 Gew.-% beträgt, bezogen auf das Gesamtgewicht der Zubereitungen, um kosmetische Zubereitungen zur Verfügung zu stellen, welche die Haut vor dem gesamten Bereich der ultravioletten Strahlung schützen.
Der größte Teil der Lichtschutzmittel in den zum Schutz der menschlichen Epidermis dienenden kosmetischen oder dermatologischen Zubereitungen besteht aus Verbindungen, die UV-Licht im UV-B-Bereich absorbieren. Beispielsweise beträgt der Anteil der erfindungsgemäß zu verwendenden UV-A-Absorber 10 bis 90 Gew.-%, be-vorzugt 20 bis 50 Gew.-% bezogen auf die Gesamtmenge von UV-B und UV-A absor-bierenden Substanzen.

### Perlglanzwachse

Geeignete Perlglanzwachse für die kosmetischen Zubereitungen sind beispielsweise auf Seite 50, Zeile 1 bis Zeile 16 der WO 2006/106140 beschrieben. Auf den Inhalt der genannten Textstelle wird hiermit in vollem Umfang Bezug genommen.
Die kosmetischen Zubereitungen können weiterhin Glitterstoffe und/oder andere Effektstoffe (z.B. Farbschlieren) enthalten.

### Emulgatoren

Die kosmetischen Zubereitungen liegen in einer bevorzugten Ausführungsform der Erfindung in Form von Emulsionen vor. Die Herstellung solcher Emulsionen erfolgt nach bekannten Methoden. Geeignete Emulgatoren für die kosmetischen Zubereitungen sind beispielsweise auf Seite 50, Zeile 18 bis Seite 53, Zeile 4 der WO 2006/106140 beschrieben. Auf den Inhalt der genannten Textstelle wird hiermit in vollem Umfang Bezug genommen.

### Parfümöle

Sollen den kosmetischen Zubereitungen Parfumöle zugesetzt werden, so sind geeignete Parfümöle beispielsweise auf Seite 53, Zeile 10 bis Seite 54, Zeile 3 der WO 2006/106140 beschrieben. Auf den Inhalt der genannten Textstelle wird hiermit in vollem Umfang Bezug genommen.

### Pigmente

Gegebenenfalls enthalten die kosmetischen Zubereitungen weiterhin Pigmente. Die Pigmente liegen im Produkt meist in ungelöster Form vor und können in einer Menge von 0,01 bis 25 Gew.%, besonders bevorzugt von 5 bis 15 Gew.% enthalten sein. Die bevorzugte Teilchengröße beträgt 1 bis 200 µm, insbesondere 3 bis 150 µm, besonders bevorzugt 10 bis 100 µm.
Geeignete Pigmente für die kosmetischen Zubereitungen sind beispielsweise auf Seite 54, Zeile 5 bis Seite 55, Zeile 19 der WO 2006/106140 beschrieben. Auf den Inhalt der genannten Textstelle wird hiermit in vollem Umfang Bezug genommen.

### Polymere

Die kosmetischen Zubereitungen enthalten in einer besonders bevorzugten Ausführungsform Polymere.
Geeignete zusätzliche Polymere für die kosmetischen Zubereitungen sind beispielsweise auf Seite 55, Zeile 21 bis Seite 63, Zeile 2 der WO 2006/106140 beschrieben. Auf den Inhalt der genannten Textstelle wird hiermit in vollem Umfang Bezug genommen.

### Beispiele

Die Erfindung wird durch die folgenden Beispiele veranschaulicht, nicht aber auf sie beschränkt.

### Beispiel 1.

### Herstellung einer Suspension von nanopartikulärem ZnO in 1,2-Propandiol

Eine Mischung von 100 g Zinkacetatdihydrat und 1000 g 1,2-Propandiol wurde unter Rühren (350 upm) innerhalb von 15 Minuten an Luft auf 100°C erhitzt.
Nach dem Erreichen der Temperatur von 100°C wurden 20 ml Wasser zugegeben, die Mischung wurde auf 150°C aufgeheizt, 30 Minuten unter Rückfluss und weitere 30 Minuten ohne Rückfluss bei dieser Temperatur gehalten und anschließend auf Raumtemperatur abgekühlt.
In einer Crossflow-Ultrafiltrations-Laboranlage (Fa. Sartorius, Typ SF Alpha, PES-Kassette, Cut off 100 kD) wurde der flüssige Anteil der erhaltenen Suspension gegen reines 1,2-Propandiol ausgetauscht. Der Anteil an Zinkoxid betrug ca. 2 Gew.-%.
Zur Bestimmung der Teilchengrößenverteilung (TGV) mittels dynamischer Lichtstreuung (Nanotrac U20591, Fa. Microtrac Inc.) wurde die erhaltene ZnO-Suspension auf ca. 0,02 Vol.-% verdünnt und in einem Ultraschallbad (Sonorex Super 10P, Fa. Bandelin) 5 Minuten bei 450 W Leistung behandelt. In einem TGV-Spektrum wies die ZnO-Suspension einen mittleren Wert (Vol.-%) für die Teilchengröße von ca. 0,18 Mikrometern auf.

### Vergleichsbeispiel 1

### Herstellung einer Suspension von nanopartikulärem ZnO in 1,2-Propandiol mit zwischenzeitlicher Lösungsmittelentfernung

Eine Mischung von 100 g Zn-Acetatdihydrat und 1000 g 1,2-Propandiol wurde unter Rühren (350 upm) innerhalb von 15 Minuten an Luft auf 100°C erhitzt.
Nach dem Erreichen der Temperatur von 100°C wurden 20 ml Wasser zugegeben, die Mischung wurde auf 150°C aufgeheizt, 30 Minuten unter Rückfluss und weitere 30 Minuten ohne Rückfluss bei dieser Temperatur gehalten und anschließend auf Raumtemperatur abgekühlt.
Die entstehende Suspension wurde in einer Zentrifuge Typ Sorvall RC-6 der Fa. Thermo bei 13000 upm zentrifugiert. Das abgesetzte ZnO-Pulver wurde von 1,2-Propandiol abgetrennt, 2 mal in Ethanol redispergiert und anschließend bei ca. 50°C während 5 Stunden in einem Trockenschrank getrocknet.
Die Röntgenbeugungsaufnahme des erhaltenen Pulvers bestätigte die Entstehung von kristallinem ZnO.

Zur Bestimmung der Teilchengrößenverteilung (TGV) mittels statischer Lichtstreuung (Mastersizer 2000, Fa. Malvern) wurde das nach dem Trocknen erhaltene ZnO-Pulver in 1,2-Propandiol redispergiert (ZnO-Gehalt ca. 2 Gew.-%) auf ca. 0,02 Vol.-% verdünnt und anschließend in einem Ultraschallbad (Sonorex Super 10P, Fa. Bandelin) 5 Minuten bei 450 W Leistung behandelt. In einem TGV-Spektrum wies die ZnO-Suspension eine agglomerierte Mikrostruktur mit einem mittleren Wert (Vol.-%) von ca. 42 Mikrometern auf.

### Anwendungsbeispiele

### Herstellung einer UV-schützenden kosmetischen Formulierung auf Basis der ZnO-Suspension in 1,2-Propandiol

### Anwendungsbeispiel 1

Die ca. 2 Gew.-%ige ZnO-Suspension aus Beispiel 1 wurde durch Absetzenlassen des ZnO und Abtrennung des überstehenden Lösemittels auf ca. 60 Gew.-% ZnO aufkonzentriert.
Die W/O-Emulsion wurde anschließend analog dem Beispiel 10 von US 6 171 580 B1 hergestellt.

### Anwendungsbeispiel 2

Die ca. 2 Gew.-%ige ZnO-Suspension aus Beispiel 1 wurde durch Absetzenlassen des ZnO und Abtrennung des überstehenden Lösemittels auf ca. 55,5 Gew.-% ZnO aufkonzentriert (Phase B).

| | Gew.-% | Bestandteile | INCI |
|---|---|---|---|
| A | 7,50 | Uvinul^{®}MC 80 | Ethylhexyl Methoxycinnamate |
| | 1,50 | Tween^{®}20 | Polysorbate-20 |
| | 3,00 | Pationic^{®}138 C | Sodium Lauroyl Lactylate |
| | 1,00 | Cremophor^{®}CO 40 | PEG-40 Hydrogenated Castor Oil |
| | 1,00 | Cetiol^{®}SB 45 | Butyrospermum Parkii (Shea Butter) |
| | 6,50 | Finsolv^{®}TN | C12-15 Alkyl Benzoate |
| B | 9,00 | Zinkoxid + 1,2-Propandiol (5 / 4) | Zinc Oxide 1,2-Propandiol |
| C | 1,00 | D-Panthenol 50 P | Panthenol, Propylene Glycol |
| | 0,30 | Keltrol^{®} | Xanthan Gum |
| | 0,10 | Edeta^{®}BD | Disodium EDTA |
| | 2,00 | Urea | Urea |
| | 2,00 | Simulgel^{®}NS | Hydroxyethyl Acrylate/Sodium Acryloyldimethyl Taurate Copolymer, Squalane, Polysorbate 60 |
| | 64,10 | Water dem. | Aqua dem. |
| D | 0,50 | Lactic Acid | Lactic Acid |
| | 0,50 | Euxyl^{®}K 300 | Phenoxyethanol, Methylparaben, Butylparaben, Ethylparaben, Propylparaben, Isobutylparaben |

Phase A wurde auf 80°C erhitzt, danach wurde Phase B zugegeben, das Gemisch wurde 3 Minuten homogenisiert. Getrennt wurde Phase C auf 80°C erhitzt und in das Gemisch von den Phasen A und B eingerührt. Das Gemisch wurde dann auf 40°C unter Rühren abgekühlt, danach wurde Phase D zugegeben. Die Lotion wurde kurz nachhomogenisiert.

### Anwendungsbeispiel 3

Die Dispersion von ZnO in Propandiol wird zu einer Wasser-in-Silikon-Formulierung zugegeben:

| Gew.-% | Inhaltsstoffe | INCI |
|---|---|---|
| Phase A | | |
| 25,0 | Dow Corning 345 Fluid | Cyclopentasiloxane, Cyclohexasiloxane |
| 20,0 | Luvitol^{™} Lite | Cyclopentasiloxane |
| 8,0 | Uvinul^{©} MC 80 | Ethylhexyl Methoxycinnamate |
| 4,0 | Abil^{©} EM 90 | Cetyl PEG/PPG-10/1 Dimethicone |
| 7,0 | T-Lite^{™} SF | Titanium Dioxide (and) Aluminum Hydroxide (and) Dimethicone/Methicone Copolymer |
| | | |

| Phase B | | |
|---|---|---|
| 17,0 | Ethanol 95 % | Alcohol |
| 9,0 | Zinkoxid + 1,2-Propandiol (5/4) | Zinc Oxide 1,2-Propandiol |
| 5,0 | Wasser dem. | Aqua dem. |
| 3,0 | Glycerin 87% | Glycerin |
| 1,0 | Talcum (C/2S Fa. Bassermann) | Talc |

Phase A und B werden bei ca. 11000 Upm für 3 Minuten homogenisiert, danach wird B zu A gegeben und für eine weitere Minute homogenisiert.

### Beispiel 4

| A | | |
|---|---|---|
| 7,00 | Uvinul^{®}MC 80 | Ethylhexyl Methoxycinnamate |
| 2,00 | Uvinul^{®}A Plus | Dimethylamino Hydroxybenzoyl Hexyl Benzoate |
| 5,00 | Uvinul^{®}N 539 T | Octocrylene |
| 3,00 | Octylsalicylat | Octyl Salicylate |
| 3,00 | Homomenthylsalicylate | Homosalate |
| | | |
| 2,00 | Antaron^{®}V-216 | PVP/Hexadecene Copolymer |
| 0,50 | Abil^{®}350 | Dimethicone |
| 0,10 | Oxynex^{®}2004 | BHT, Ascorbyl Palmitate, Citric Acid, Glyceryl Stearate, Propylene Glycol |
| 2,00 | Cetylalkohol | Cetyl Alcohol |
| 2,00 | Amphisol^{®}K | Potassium Cetyl Phosphate |
| | | |

| B | | |
|---|---|---|
| 3,00 | Zinkoxid + 1,2-Propandiol | |
| 5,00 | 1,2-Propylenglykol Care | Propylene Glycol |
| 57,62 | Water | Aqua dem. |
| 0,20 | Carbopol^{®}934 | Carbomer |
| 5,00 ' | Witconol^{®}APM | PPG-3 Myristyl Ether |

| C | | |
|---|---|---|
| 0,50 | Euxyl^{®}K300 | Phenoxyethanol, Methylparaben, Ethylparaben, Ethylparaben, Butylparaben, Propylparaben and Isobutylparaben |

### Herstellung:

Phase A wird bis zum Schmelzen bei ca. 80°C erhitzt und für ca. 3 min homogenisiert;
Phase B wird ebenfalls bis ca. 80°C erhitzt, zu Phase A gegeben und diese Mischung wird wiederum homogenisiert. Danach wird unter Rühren bis auf Raumtemperatur abkühlen gelassen. Danach wird Phase C zugegeben und wiederum homogenisiert.

## Patentansprüche

1. Verfahren zur Herstellung von Metalloxid enthaltenden kosmetischen Zubereitungen umfassend wenigstens folgende Schritte:
a) Herstellung des Metalloxids durch Umsetzung einer geeigneten Vorstufe in einer Alkohol umfassenden Reaktionsmischung,
b) gegebenenfalls Entfernen von bis zu 90 Gew.-% der flüchtigen Bestandteile der aus Schritt a) erhaltenen Metalloxid-Reaktionsmischung,
c) gegebenenfalls wenigstens teilweises Austauschen der flüssigen Phase 1 der Reaktionsmischung gegen eine von der flüssigen Phase 1 unterschiedliche flüssige Phase 2,
d) Verwendung der nach den Schritten a) bis c) erhaltenen Reaktionsmischung zur Herstellung der kosmetischen Zubereitung.

2. Verfahren nach Anspruch 1), **dadurch gekennzeichnet, dass** das Metalloxid in Form von Teilchen mit einer zahlenmittleren Teilchengröße von weniger als 1000 Nanometern vorliegt.

3. Verfahren nach einem der Ansprüche 1)oder 2), **dadurch gekennzeichnet, dass** das Metalloxid in Form von Teilchen mit einer zahlenmittleren Teilchengröße von weniger als 500 Nanometern vorliegt.

4. Verfahren nach einem der Ansprüche 1) bis 3), wobei das Metalloxid Zinkoxid ist.

5. Verfahren nach einem der Ansprüche 1) bis 4), **dadurch gekennzeichnet, dass** der Alkohol ausgewählt ist aus Alkoholen mit wenigstens zwei OH-Gruppen.

6. Verfahren nach einem der Ansprüche 1) bis 5), **dadurch gekennzeichnet, dass** der Alkohol ausgewählt ist aus der Gruppe bestehend aus 1,2-Ethandiol, 1,2-Propandiol und deren Mischungen.

7. Verfahren nach einem der Ansprüche 1) bis 6), **dadurch gekennzeichnet, dass** die nach Schritt a) erhaltene Reaktionsmischung im Bereich von 30 bis 99 Gew.-% Alkohol enthält.

8. Verfahren nach einem der Ansprüche 1) bis 7), **dadurch gekennzeichnet, dass** die in Schritt a) verwendete geeignete Vorstufe ausgewählt ist unter Verbindungen der allgemeinen Formel I
Zn(O)ₚ(OCOR)ₓ(OH)_{y}(OR')_{z} (I)
wobei
R H, Alkyl, Cycloalkyl, Aryl oder Arylalkyl bedeutet,
R' Alkyl, Cycloalkyl, Aryl oder Arylalkyl bedeutet,
p = (2-x-y-z)/2,
x+y+z ≤ 2,
0<x≤2,
0≤y<2,
0≤z<2.

9. Verfahren nach einem der Ansprüche 1) bis 8), **dadurch gekennzeichnet, dass** die in Schritt a) verwendete geeignete Vorstufe Zinkacetatdihydrat Zn(OCOCH₃)₂*2H₂O ist.

10. Verfahren nach einem der Ansprüche 1) bis 9), **dadurch gekennzeichnet, dass** die Reaktionsmischung in Schritt a) nacheinander auf zwei verschiedene Temperaturen T1 und T2 im Bereich von 70 bis 200°C erwärmt wird, wobei T2 größer ist als T1.

11. Verfahren nach einem der Ansprüche 1) bis 10), **dadurch gekennzeichnet, dass** Schritt c) eine Ultrafiltration umfasst.

12. Verfahren nach einem der Ansprüche 1) bis 11), **dadurch gekennzeichnet, dass** die kosmetische Zubereitung ein hautkosmetisches UV-Lichtschutzmittel ist.

13. Verfahren nach einem der Ansprüche 1) bis 12), **dadurch gekennzeichnet, dass** die kosmetische Zubereitung wenigstens einen organischen UV-Lichtschutzfilter enthält.

14. Verfahren nach einem der Ansprüche 1) bis 13), **dadurch gekennzeichnet, dass** die kosmetische Zubereitung wenigstens ein Antioxidationsmittel enthält.

15. Verfahren nach einem der Ansprüche 1) bis 14), **dadurch gekennzeichnet, dass** die kosmetische Zubereitung wenigstens ein Selbstbräunungsmittel enthält.

16. Verfahren nach einem der Ansprüche 1) bis 15), **dadurch gekennzeichnet, dass** die kosmetische Zubereitung wenigstens einen weiteren Alkohol und/oder wenigstens ein Öl enthält.

17. Verfahren nach einem der Ansprüche 1) bis 16), **dadurch gekennzeichnet, dass** die kosmetische Zubereitung wenigstens einen weiteren anorganischen UV-Lichtschutzfilter enthält.

18. Verfahren nach einem der Ansprüche 1) bis 17), **dadurch gekennzeichnet, dass** die kosmetische Zubereitung weiterhin wenigstens einen Acrylsäure-Verdicker enthält.
